# EUROPEAN PATENT SPECIFICATION

(11) **EP 3 342 432 B1**
(45) Date of publication and mention of the grant of the patent: **07.07.2021**
(21) Application number: 17202084.4
(22) Date of filing: 03.08.2010
(51) Int. Cl.: A61L 27/36

(54) **BONE MATRIX COMPOSITIONS AND METHODS**
KNOCHENMATRIX-ZUSAMMENSETZUNGEN UND -VERFAHREN
COMPOSITIONS DE MATRICE OSSEUSE ET PROCÉDÉS AFFÉRENTS

(30) Priority: 03.08.2009 US 230890 P; 18.02.2010 US 305745 P
(43) Date of publication of application: 04.07.2018
(62) Divisional of application: 10742963.1
(73) Proprietor: Warsaw Orthopedic, Inc., Warsaw, IN 46581 (US)
(72) Inventor: WEI, Guobao, Milltown, NJ 08850 (US); SHIMP, Lawrence A., Morganville, NJ 07751 (US); DEAN, Sheldon W., III, Jackson, NJ 08527 (US); DECARO, Mark, Millstone Township, NJ 08535 (US); SCHWARZ, Carl R., Lincroft, NJ 07738 (US); OUKO, Martin O., Somerset, NJ 08873 (US); BEHNAM, Keyvan, Simi Valley, CA 93065 (US)
(74) Representative: FRKelly

(56) References cited:
- WO-A1-98/41245
- WO-A2-2008/157492
- WO-A2-2008/157495
- WO-A2-2009/082554

## Description

### CROSS REFERENCE TO RELATED APPLICATIONS

This application claims benefit under 35 U.S.C. § 119(e) of priority to U.S. Provisional Application No. 61/230,890 filed August 3, 2009, entitled "Bone Matrix Compositions and Methods," and U.S. Provisional Application No. 61/305,745, filed February 18, 2010, entitled "Bone Matrix Compositions and Methods".

### BACKGROUND

### Introduction

Mammalian bone tissue is known to contain one or more proteinaceous materials, presumably active during growth and natural bone healing, that can induce a developmental cascade of cellular events resulting in endochondral bone formation. Various factors are present in bone. These include bone morphogenetic or morphogenic proteins (BMPs), bone inductive proteins, bone growth or growth factors, osteogenic proteins, or osteoinductive proteins. While these factors have different effects and functions, for the purposes of discussion, these will be referred to collectively herein as osteoinductive factors.

It is known that bone contains these osteoinductive factors. These osteoinductive factors are present within the compound structure of cortical bone and are present at very low concentrations, e.g., 0.003%. Osteoinductive factors direct the differentiation of pluripotent mesenchymal cells into osteoprogenitor cells that form osteoblasts. Based upon the work of Marshall Urist as shown in U.S. Pat. No. 4,294,753, issued Oct. 13, 1981, proper demineralization of cortical bone exposes the osteoinductive factors, rendering it osteoinductive, as discussed more fully below.

### Overview of Bone Grafts

The rapid and effective repair of bone defects caused by injury, disease, wounds, or surgery has long been a goal of orthopaedic surgery. Toward this end, a number of compositions and materials have been used or proposed for use in the repair of bone defects. The biological, physical, and mechanical properties of the compositions and materials are among the major factors influencing their suitability and performance in various orthopaedic applications.

Autologous cancellous bone ("ACB") long has been considered the gold standard for bone grafts. ACB includes osteogenic cells, which have the potential to assist in bone healing, is nonimmunogenic, and has structural and functional characteristics that should be appropriate for a healthy recipient. Some people do not have adequate amounts of ACB for harvesting. These people include, for example, older people and people who have had pervious surgeries. A majority of people however do have adequate amounts of ACB for harvesting. There may nevertheless be reluctance to harvest because of pain at the harvest site and donor site morbidity.

Much effort has been invested in the identification and development of alternative bone graft materials. Urist has published seminal articles on the theory of bone induction and a method for decalcifying bone, i.e., making demineralized bone matrix (DBM). Urist M.R., Bone Formation by Autoinduction, Science 1965; 150(698):893-9; Urist M.R. et al., The Bone Induction Principle, Clin. Orthop. Rel. Res. 53:243-283, 1967. As mentioned above, DBM is an osteoinductive material, in that it induces bone growth when implanted in an ectopic site of a rodent, owing to the osteoinductive factors contained within the DBM. Honsawek et al. (2000). It is now known that there are numerous osteoinductive factors, e.g., BMP 1-18, which are part of the transforming growth factor-beta (TGF-beta) superfamily. BMP-2 has been widely studied. There are also other proteins present in DBM that are not osteoinductive alone but still contribute to bone growth, including fibroblast growth factor-2 (FGF-2), insulin-like growth factor-I and -II (IGF-I and IGF-II), platelet derived growth factor (PDGF), and transforming growth factor-beta 1 (TGF-beta.1) (Hauschka, et al. 1986; Canalis, et al, 1988; Mohan et al. 1996). Moreover, WO2009082554 relates generally to bone matrix compositions and, more specifically, to bone matrix compositions having nanoscale textured surfaces and methods for their production.

Various cocktails of growth factors have been measured in DBM, including BMP2, TGFβ1, FGFa, IGF-I, PDGF, VEGF (Wildemann et al, 2007), BMP4 (Blum et al, 2004 and Honsaweket et al, 2005), and BMP7 (Pietrzak et al, 2006). Other extracellular matrix proteins have also been measured including type I collagen, fibronection, Bone Sialoprotein (BSP), and osteopontin (Shigeyama et al, 1995). Combinations of growth factors may be more osteoinductive than a single growth factor (Kawai et al, 2006, Mehlhorn et al, 2007, Shintani et al, 2007, Raiche et al, 2004 and Ripamonti et al, 1997). DBM extracellular matrix proteins such as collagen work as a natural carrier for the growth factors (Reddi et al, 2000).

DBM implants have been reported to be particularly useful (see, for example, U.S. Patent Nos. 4,394,370, 4,440,750, 4,485,097, 4,678,470, and 4,743,259; Mulliken et al., Calcif Tissue Int. 33:71, 1981; Neigel et al., Opthal. Plast. Reconstr. Surg. 12:108, 1996; Whiteman et al., J. Hand. Surg. 18B:487, 1993; Xiaobo et al., Clin. Orthop. 293:360, 1993). Useful DBM implants are disclosed in U.S. Patent Nos. 5,073,373; 5,284,655; 5,290,558; 5,314,476; 5,507,813; 5,510,396; and 5,676,146. DBM typically is derived from cadavers. The bone is removed aseptically and treated to kill any infectious agents. The bone is typically particulated by milling or grinding, and then the mineral component is extracted by various methods, such as by soaking the bone in an acidic solution. The remaining matrix is malleable and can be further processed and/or formed and shaped for implantation into a particular site in the recipient. Demineralizing bone makes available and exposes active growth factors in the bone matrix. Following implantation, the presence of DBM induces cellular recruitment to the site of injury. The recruited cells may eventually differentiate into bone forming cells. Such recruitment of cells leads to an increase in the rate of wound healing and, therefore, to faster recovery for the patient.

There are several issues to consider with respect to processing methods for producing bone compositions such as DBM. Typical processes for forming DBM involve two steps: a demineralization step and a separate particulation step. The shape of the particles formed in the process, for example whether fibrous particles (or fibers) or generally spherical particles, may affect osteoinductivity of the DBM.

Bone has a complex hierarchical structure. Bones generally comprise an external shell of cortical bone and a spongy interior of cancellous bone. Cortical bone is often built out of lamellae comprising mineralized collagen fibrils. The basic material of bone is a collagen-mineral composite comprising nano-sized mineral platelets (essentially carbonated hydroxyapatite), protein (predominately collagen type I), and water. Both the organic and mineral component of bone, as well as their geometric arrangement, contribute to bone strength. Fratzl et al., Structure and Mechanical Quality of the Collagen-Mineral Nano-Composite in Bone, Journal of Materials Chemistry, 2004.

Bone material comprises collagen fibrils reinforced with tiny mineral particles, a few nanometers in thickness. The mineral particles are typically aligned with the collagen matrix. The organic matrix of bone comprises collagen and a series of non-collagenous proteins and lipids. Some 85-90% of the total bone protein comprises collagen fibers. Type I collagen is a largely fibrous protein with a repetitive amino acid sequence, which allows three polypeptide chains to fold into a unique triple-helical structure. Collagen's intermolecular cross-linking provides the fibrillar matrices with various mechanical properties such as tensile strength and viscoelasticity. Native collagen type I is a hydrated moiety and greatly contributes to the viscoelastic proerties of bone. The alteration of collagen structural properties has been found to be a factor contributing to reduced mechanical properties of bone. Fratzl et al., Structure and Mechanical Quality of the Collagen-Mineral Nano-Composite in Bone, Journal of Materials Chemistry, 2004.

Given the natural alignment of collagen structure and the importance of the collagen structure to the properties of bone, particulation of bone along the natural separation lines between the collagen structures leads to increased preservation of the natural properties ofbone.

The natural properties of bone, and, more particularly, the osteoinductive factors present in bone, can be affected by the processing methods used. Osteoinductive factors can be affected in demineralization methods for making DBM. In some processing methods, osteoinductive factors may be inactivated or their activity diminished. Thus, in some cases, a bone graft may be less inductive or may be non-inductive after processing. Generally, the order of treatment, the time of acid and/or alcohol exposure, and other process parameters can impact maintenance of natural osteoinductivity of bone through processing. The processing method thus can affect the potential of the bone graft for biological activity.

A one-step method that demineralizes while separating the bone can have advantages over other methods, by saving time, maintaining biological activity of bone, increasing yield of produced bone, and/or reducing contamination during processing. Further, a processing method that forms fibers during demineralization may be advantageous by maintaining natural structures. Thus, it would be useful to provide methods for producing bone compositions that protect the biological activity of the bone.

### SUMMARY

Methods of forming demineralized bone fibers and the formed bone fibers are disclosed. It is to be appreciated that some aspects of the methods described herein may be utilized in forming demineralized bone of a non-fibrous nature. Thus, while the description herein generally relates to bone fibers, for the purposes of illustration only, this disclosure is not limited to bone fibers, and includes bone particles of any desired or suitable dimension and shape, including powders, chips, fibers, etc. According to the invention, there is provided a method for demineralizing bone comprising: demineralizing the bone in a demineralization medium, applying a pulsating vacuum to the demineralization medium during demineralizing, and separating the bone into particles during demineralization.

The method may include separating bone into fibers and demineralizing the bone wherein separating and demineralizing are done concurrently. Demineralizing the bone may be done at a low temperature.

The method may include demineralizing the bone in a demineralization medium that is treated while the bone is in the demineralization medium such that the demineralization is enhanced or optimized. The treatment may reduce the temperature of the demineralization medium and/or increase the rate of demineralization.

The thus formed demineralized fibers may be formed into an osteoimplant and may be applied at a bone repair site, or a site for bone augmentation or ectopic bone formation (e.g., lateral spine fusion). Examples include a site resulting from injury, defect brought about during the course of surgery, infection, malignancy or developmental malformation. The osteoinductive compositions may also be used as drug delivery devices.

This application refers to various patents, patent applications, journal articles, and other publications: PCT/US04/43999; PCT/US05/003092; US 2003/0143258 A1; PCT/US02/32941; Current Protocols in Molecular Biology, Current Protocols in Immunology, Current Protocols in Protein Science, and Current Protocols in Cell Biology, John Wiley & Sons, N.Y., edition as of July 2002; Sambrook, Russell, and Sambrook, Molecular Cloning: A Laboratory Manual, 3rd ed., Cold Spring Harbor Laboratory Press, Cold Spring Harbor, 2001; Rodd 1989 "Chemistry of Carbon Compounds," vols. 1-5 and supps, Elsevier Science Publishers, 1989; "Organic Reactions," vols 1-40, John Wiley and Sons, New York, NY, 1991; March 2001, "Advanced Organic Chemistry," 5th ed. John Wiley and Sons, New York, NY. Where numerical values herein are expressed as a range, endpoints are included.

While multiple embodiments are disclosed, still other embodiments of the present invention will become apparent to those skilled in the art from the following detailed description. Accordingly, the detailed description is to be regarded as illustrative in nature and not restrictive. The scope of the invention is set out in the claims attached hereto.

### BRIEF DESCRIPTION

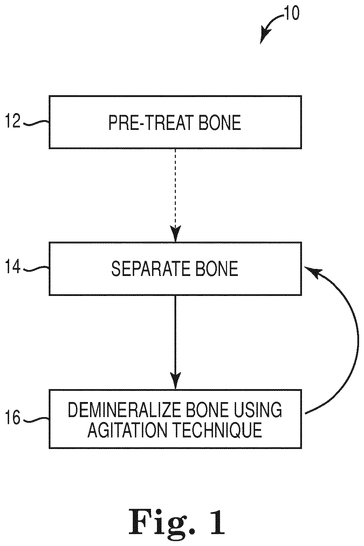

Figure 1 illustrates a method of forming a demineralized bone matrix in accordance with one embodiment.

### DEFINITIONS

*Bioactive Agent* or *Bioactive Compound* is used herein to refer to a compound or entity that alters, inhibits, activates, or otherwise affects biological or chemical events. For example, bioactive agents may include, but are not limited to, osteogenic or chondrogenic proteins or peptides, anti-AIDS substances, anti-cancer substances, antibiotics, immunosuppressants, anti-viral substances, enzyme inhibitors, hormones, neurotoxins, opioids, hypnotics, anti-histamines, lubricants, tranquilizers, anti-convulsants, muscle relaxants and anti-Parkinson substances, anti-spasmodics and muscle contractants including channel blockers, miotics and anti-cholinergics, anti-glaucoma compounds, anti-parasite and/or anti-protozoal compounds, modulators of cell-extracellular matrix interactions including cell growth inhibitors and antiadhesion molecules, vasodilating agents, inhibitors of DNA, RNA or protein synthesis, anti-hypertensives, analgesics, anti-pyretics, steroidal and non-steroidal anti-inflammatory agents, anti-angiogenic factors, angiogenic factors, anti-secretory factors, anticoagulants and/or antithrombotic agents, local anesthetics, ophthalmics, prostaglandins, anti-depressants, anti-psychotic substances, anti-emetics, and imaging agents. In certain disclosures, the bioactive agent is a drug. Bioactive agents further include RNAs, such as siRNA, and osteoclast stimulating factors. In some disclosures, the bioactive agent may be a factor that stops, removes, or reduces the activity of bone growth inhibitors. In some disclosures the bioactive agent is a growth factor, cytokine, extracellular matrix molecule or a fragment or derivative thereof, for example, a cell attachment sequence such as RGD. A more complete listing of bioactive agents and specific drugs suitable for use in the present invention may be found in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", Edited by Susan Budavari et al., CRC Press, 1996; and the United States Pharmacopeia-25/National Formulary-20, published by the United States Pharmcopeial Convention, Inc., Rockville MD, 2001.

*Biocompatible,* as used herein, is intended to describe materials that, upon administration *in vivo,* do not induce undesirable long-term effects.

*Bone* as used herein refers to bone that is cortical, cancellous or corticocancellous of autogenous, allogenic, xenogenic, or transgenic origin.

*Bone particles* herein refers to relatively small bone pieces such as fibers, bundles of loosely connected fibers, threads, narrow strips, thin sheets, chips, shards, powders, other shapes, etc., that possess regular, irregular, or random geometries and which can be separated form each other to any desired extent. While description herein may be made specifically to bone fibers in some embodiments, as noted it is to be appreciated that bone fibers are merely one type of particle that may be formed using methods as disclosed herein.

*Demineralized,* as used herein, refers to any material generated by removing mineral material from tissue, *e.g.,* bone tissue. In certain disclosures, the demineralized compositions described herein include preparations containing less than 5% calcium and preferably less than 1% calcium by weight. Partially demineralized bone (*e.g.,* preparations with greater than 5% calcium by weight but containing less than 100% of the original starting amount of calcium) is also disclosed. In some disclosures, demineralized bone has less than 95% of its original mineral content. Demineralized is intended to encompass such expressions as "substantially demineralized," "partially demineralized," and "fully demineralized."

*Demineralized bone matrix,* as used herein, refers to any material generated by removing mineral material from bone tissue. In some disclosures, the DBM compositions as used herein include preparations containing less than 5% calcium and preferably less than 1% calcium by weight. Partially demineralized bone (*e.g.,* preparations with greater than 5% calcium by weight but containing less than 100% of the original starting amount of calcium) are also disclosed.

*Osteoconductive* is used herein to refer to the ability of a non-osteoinductive substance to serve as a suitable template or substance along which bone may grow.

*Osteogenic* is used herein to refer to the ability of an agent, material, or implant to enhance or accelerate the growth of new bone tissue by one or more mechanisms such as osteogenesis, osteoconduction, and/or osteoinduction.

*Osteoimplant* as used herein refers to any bone-derived implant prepared in accordance with the disclosures of this invention and therefore is intended to include expressions such as bone membrane, bone graft, etc.

*Osteoinductive,* as used herein, refers to the quality of being able to recruit cells from the host that have the potential to stimulate new bone formation. Any material that can induce the formation of ectopic bone in the soft tissue of an animal is considered osteoinductive. For example, most osteoinductive materials induce bone formation in athymic rats when assayed according to the method of Edwards et al., "Osteoinduction of Human Demineralized Bone: Characterization in a Rat Model," Clinical Orthopaedics & Rel. Res., 357:219-228, December 1998. In other instances, osteoinduction is considered to occur through cellular recruitment and induction of the recruited cells to an osteogenic phenotype. *Osteoinductivity* score refers to a score ranging from 0 to 4 as determined according to the method of Edwards *et al.* (1998) or an equivalent calibrated test. In the method of Edwards *et al.,* a score of "0" represents no new bone formation; "1" represents 1%-25% of implant involved in new bone formation; "2" represents 26-50% of implant involved in new bone formation; "3" represents 51%-75% of implant involved in new bone formation; and "4" represents >75% of implant involved in new bone formation. In most instances, the score is assessed 28 days after implantation. However, the osteoinductivity score may be obtained at earlier time points such as 7, 14, or 21 days following implantation. In these instances it may be desirable to include a normal DBM control such as DBM powder without a carrier, and if possible, a positive control such as BMP. Occasionally osteoinductivity may also be scored at later timepoints such as 40, 60, or even 100 days following implantation. Percentage of osteoinductivity refers to an osteoinductivity score at a given time point expressed as a percentage of activity, of a specified reference score.

### DETAILED DESCRIPTION

### I. INTRODUCTION

The present invention provides a method for demineralizing bone comprising: demineralizing the bone in a demineralization medium, applying a pulsating vacuum to the demineralization medium during demineralizing, and separating the bone into particles during demineralization.

As shown in Figure 1, in one embodiment, the method 10 may include pretreating the bone [block 12], separating the bone [block 14], and demineralizing the bone [block 16], wherein demineralizing the bone may include treating the demineralization medium to enhance or optimize demineralization. Separating the bone may be done contemporaneously with demineralizing the bone. The bone also may be separated before; before and during; before, during, and after; during and after; and/or after demineralization, as taught herein. Further, multiple demineralization steps and/or multiple separation steps may be used. For example, in some embodiments, separating the bone may be done by applying a force to the bone and, in further embodiments, multiple applications of force may be done such as a pre-crush or a post-demineralization separation. Those of ordinary skill will appreciate that a variety of embodiments or versions of the invention are not specifically discussed below but are nonetheless defined by the appended claims.

Using methods such as disclosed herein, bone fibers are provided that substantially retain their natural structure and morphology as well as their biological activity. Methods provided herein may alternatively be used to produce bone particles of any dimension, including generally spherical particles, plates, or other. The methods provided herein include a demineralization method that decreases acid exposure time and can be performed at a low temperature that is generally favorable for protecting the biological activity of the bone. As will be explained, the demineralization methods disclosed herein provide for the replenishment of active acid in the regions adjacent the bone during the demineralization process, which regions, in the absence of the methods of the inventions disclosed herein, may become depleted, thus necessitating prolonged exposure of bone to acid during the demineralization process. The methods disclosed herein also provide for lower temperatures. Lower temperatures may aid in the preservation of the osteoinductive factors during processing, including the demineralization phase. With the presently disclosed procedure, lower temperatures may be possible without extending reaction times. This effect may be aided by increasing the surface area of the mineralized bone while also increasing penetration of the bone by demineralization medium and/or removal of reacted demineralization medium. By decreasing the amount of time for demineralization as well as the temperature of the demineralization, any detrimental effects of the demineralization process on osteoinductive factors in the bone are mitigated. More specifically, the methods reduce detrimental effects of acid (used in demineralization) on proteins in bone. The osteoinductivity of bone is thus better maintained and an osteoinductive demineralized bone matrix formed.

Bone is made up principally of collagen, cells, minerals, and other noncollagenous proteins. Bone matrices can be nondemineralized, partially demineralized (including surface demineralized), demineralized, deorganified, anorganic, or mixtures of these. Demineralized bone matrix (DBM) is comprised principally of proteins and glycoproteins, collagen being the primary protein component of DBM. While collagen is relatively stable, normally being degraded only by the relatively rare collagenase enzymes, various other proteins and active factors present in DBM are less stable, and may be quickly degraded by conditions in a standard demineralization process (such as acid and heat). Osteoinductive factors also may be degraded by exposure to enzymes naturally present in the host, such as proteases and sugar-degrading enzymes (*e.g.,* endo- and exoglycosidases, glycanases, glycolases, amylase, pectinases, galacatosidases, *etc.*). Reducing demineralization exposure time can reduce degradation of the osteoinductive factors.

### II. PROVIDE BONE

The bone useful in the invention herein may be obtained utilizing methods well known in the art, e.g., allogenic donor bone. Bone-derived elements can be readily obtained from donor bone by various suitable methods, e.g., as described in U.S. Patent No. 6,616,698. Bone suitable for making demineralized bone fibers or particles using methods disclosed herein can be bone from any source. Thus, autogenic, allogenic, xenogenic, or transgenic bone can be used. Suitable types of xenogenic bone may include porcine, equine, or bovine. The bone can be cortical, cancellous or corticocancellous. In specific disclosures described herein, the bone is cortical allogenic bone, e.g., femur, tibia, fibula, radius, ulna, etc.

The methods disclosed herein are not limited to any size or configuration of bone to be treated, and are applicable to bone in a variety of sizes. Bone utilized as the starting, or stock, material may range in size from relatively small pieces of bone to bone of such dimensions as to be recognizable as to its anatomical origin to whole bone. For example, the stock material may range from fragments to whole bone and the formed bone composition may range from particles to fibers to plates. In some disclosures, the whole bone may be partially subdivided, such as into strips, for further processing by methods disclosed herein.

### III. SEPARATE THE BONE

Separating the bone (or separation) refers to subdivision of the bone. Separating the bone may provide space and surface area for cellular activity (osteoconductivity). Separating the bone may further expose bone inducing/facilitating proteins to induce bone regeneration (osteoinductivity). Separation of the bone may be performed before, during, and/or after demineralization of the bone, as noted above. Various separation techniques are disclosed herein but it is to be appreciated that other separation techniques may be used. Generally, separation may be done in a manner such that the bone subdivides in a manner generally consistent with natural morphology. In some disclosures, separation may be done in up to three steps. An initial crush or pressing may be done to form openings or acid entry routes in the bone, generally along natural boundaries or separation planes that exist in the bone, such as the cement lines of osteons; this initial separation step may enhance demineralization efficiency when performed before and/or during demineralization. Another crush may be done to separate the bone into particle fibers. A third crush may be done to further separate the bone. In some disclosures, the second crush may be done concurrently with demineralization. These steps may be combined, as desired.

Separation may be used to subdivide the bone into bone particles, fibers, plates, or other desired configuration and size. In some embodiments, separation may be used to subdivide the bone into bone fibers. Thickness and width of the fibers may be generally equal and length of the fibers may be up to the length of the stock material.

Depending on the procedure employed for separating the bone, one can obtain a mass of bone fibers or particles in which at least about 80 weight percent, at least about 90 weight percent, or at least about 95 weight percent, of the fibers or particles possess a median length of from about 2 to about 300 mm or greater, or a median length of from about 5 to about 50 mm, a median thickness of from about 0.5 to about 15 mm, or a median thickness of from about 1 to about 5 mm, a median width of from about 2 to about 35 mm, or a median width of from about 2 to about 20 mm and a median length to thickness ratio and/or a median length to width ratio of from about 2 to 200, or from about 10 to about 100. In some disclosures, the bone fibers can be graded or sorted into different sizes, e.g., by screening, and/or any less desirable size(s) of bone fibers or particles that may be present can be reduced or eliminated, and/or sorted for use in other applications with other therapeutic products. In accordance with some examples, the bone may be separated into small fibers (selected using a 1679 µm sieve), large fibers (selected using a 2000 µm sieve), and very large fibers (selected using a 4000 µm sieve). In specific disclosures, fibers created using methods described herein may have a thickness and/or width of between approximately 0.4 and 4 mm or larger.

In some disclosures, cortical bone is treated, including separating and demineralizing, according to methods disclosed herein. A first disclosure of separation comprises producing bone fibers by separating cortical bone (such as longitudinally) under pressure. In some disclosures, separation is done during demineralization and a further separation step, such as an initial crush to form acid entry routes or a subsequent crush to further separate into smaller fibers, may be done. In alternative disclosures, other types of bone may be treated as disclosed herein.

In some embodiments separation may be done by applying a force to the bone during demineralization. As will be described more fully below, such force may be applied using a roller press, using a ball mill, using impact crushing, using pressure crushing, or otherwise applying a force manually or in an automated and/or computer-controlled fashion to the bone.

Separation may be done by any suitable technique. For example, machines or instruments that cut, shred, mill, extrude, and/or chop may be used. Examples of suitable equipment may use a sharp bit or blade on or into the bone to cut or mill the bone, including by cutting away material that is not in the desired configuration (such as material that is not fibrous). For example, mills, including ball mills, impact mills, grating mills, shearing mills, and cutting mills, may be used.

Separation also may be done using, for example, impact crushing and/or pressure crushing, which tends to separate the bone along natural boundaries or separation planes that exist in the bone. These separation planes may be parallel to the underlying collagen bundles, and include the cement lines of osteons. In various embodiments, the separation phase comprises pressing bone in a press to form fibers. A simple pressing technique comprises application of pressure to unconstrained demineralized bone, such as via mortar and pestle, by applying a rolling/pressing motion such as is generated by a rolling pin or by two or more rollers, by rollers aligned opposite a conveyor or moving surface, or by pressing the bone pieces between flat or curved plates. These techniques compress the bone, which causes mechanical deformation and rupturing of bonds between collagen fibers, or between fibrils, as these overlapping structures are less strong than the polypeptide strands or the helices. These flattening pressures cause the bone fibers to separate. The rollers act as a force to apply pressure for a defined period of time and potentially to make a continuous process out of what is traditionally a batch process. Pressing demineralized bone provides substantially intact natural bone collagen fibers that can be as long as the fibers in the demineralized bone stock from which they were obtained. In various embodiments, pressing (also referred to as crushing) may be done to a single layer of bone or to a plurality of layers ofbone.

Generally, any suitable type of roller pressing may be used. Further, various parameters of roller pressing may be varied to achieve desired results. For example, rollers may be provided running at different speeds but in the same direction, sintering may be done, a roller press may be provided that is cam shaped to load at specific points, and the substrate and/or roller may be configured to cause a bending effect as a point load.

The method of the invention is not limited to any particular way of applying force to the bone. Suitable devices for pressing bone include industrial presses, stampers, and punches. One example of a suitable press is a press that may provide a substrate or platen for receiving a bone and a plate that may be pressed to the substrate, thereby crushing the bone between the substrate and the press. In some examples, one or both of the substrate and the plate may be curved, flat, have grooves, or may be otherwise shaped. In some embodiments, treated and dried (such as pre-treated and demineralized, both described below) bone may be pressed to separate the bone. Using a pneumatic operational industrial press, such separation may be done in an hour.

Another pressing technique involves mechanically pressing demineralized bone that is constrained within a sealed chamber having a hole (or a small number of holes) in its floor or bottom plate. The separated fibers extrude through the hole or holes, with the hole diameter limiting the maximum diameter of the extruded fibers. As with unconstrained pressing methods, this constrained method results in fibers that are largely intact (as far as length is concerned) but separated bone collagen bundles.

In a combined unconstrained/constrained pressing technique that results in longer fibers by minimizing fiber breakage, the demineralized bone is first pressed into an initially separated mass of fibers while in the unconstrained condition and thereafter these fibers are constrained within a sealed chamber where pressing is continued.

In general, pressing of demineralized bone to provide demineralized bone particles can be carried out at any suitable pressure that causes the division of bone. The specific pressure applied may vary based on dimensions of the bone and processing of the bone. Accordingly, the ranges given below are intended merely as examples. The pressure need only be sufficient to separate the bone to the desired extent, and should not be so great as to cause more mechanical deformation than is desired. By way of example only, pressing may in some disclosures at pressures up to about 275.79 MPa (40,000 psi) (or higher, if desired), from about 3.44 MPa (500 psi) to about 206.84 MPa (30,000 psi), or from about 6.89 MPa (1,000 psi) to about 137.89 MPa (20,000 psi).

In further embodiments, separating the bone comprises running the bone through a rolling press such as a roller compactor. Such rolling may be done during demineralization and/or before or after demineralization. To run the bone through the roller compactor during demineralization, the bone may if desired initially be soaked with a demineralization medium such as an acid and then run through the roller press. In some disclosures, the bone may be divided into strips before being run through a roller to separate into fibers. In other embodiments, the bone may be pressed sufficiently to result in some level of separation; demineralized; and then further pressed to create additional separation.

A roller compactor generally comprises two counter rotating rolls with a gap provided between the rolls. In various disclosures, the gap may range from approximately 0.15 mm (0.006 inch) to approximately 0.36 mm (0.014 inch). In some disclosures, the rollers may be knurled rollers. The bone is run through the gap and contacted with the rollers. Any suitable pressure may be applied, including between approximately 525380 newton/m (3000 lbs/in) and approximately 1313451 newton/m (7500 lbs/in). Some of the factors influencing the force applied include roll surface, diameter, peripheral speed, separating force or pressure capabilities, and feed screw design. These may be selected such that fibers of suitable dimensions are formed after running the bone through the roller compactor. In some embodiments, a roller press may be used having a grooved roller. The direction of bone feed into the roller may be varied for specific results. For example, the bone may be fed into the roller in a direction to cause longitudinal separation in the bone. Longitudinal separation may refer to separation of the bone along the bone's natural long axis (the longitudinal axis of the bone within the body), or a long axis of a piece or fragment of bone. A longitudinal axis may be the longitudinal axis of natural bone within the body, or the longitudinal axis of a piece or fragment of bone.

In other disclosures, separating the bone comprises processing the bone through a crushing chute. The crushing chute employs impact crushing. The crushing chute may comprise a chute having a bottom substrate and an upper aperture. The bone may be placed on the bottom substrate or platen. A weight may be guided and dropped through the chute onto bone. In one disclosure, the chute may be approximately 152.4 mm (6 inches) in diameter and approximately 76.2 mm (3 inches) in length. The weight may be approximately 15.87 kg (35 pounds). The size of the chute and the weight of the weight may be varied depending on the size of the bone to be separated.

In yet other embodiments, separating may be done using a combination of various separation techniques. In other disclosures, separating the bone may be done based on temperature effects. For example, in one disclosure, the bone may be frozen and fragmented in a frozen state. For example, the bone may be dipped into liquid nitrogen and then impacted with a hammer or other impact element.

In other disclosures, separating the bone comprises placing the bone in a centrifuge with a weight on top and spinning the bone with a high g-force. The addition of the g-force combined with the weight presses the tissue in between the weight on top and the bottom of the centrifuge vessel. Such spinning may be done before, during, or after demineralization. In certain embodiments, a demineralization medium may be placed in the centrifuge with the bone such that demineralization may occur in the centrifuge. Using a weight on top of the bone before or during the early stages of demineralization causes an initial breaking of the bone and a weight on top of the bone as demineralization continues facilitates separation of the bone into fibers. Generally, spinning or centrifuging accelerates acid penetration and, thus, demineralization rate, thereby decreasing demineralization time. Centrifuging may be done in a sealed environment. In some embodiments, a vacuum may be applied during centrifugation. Generally, centrifugation provides the benefits of potentially applying force via a weight on top during demineralization, applying vacuum, as well as controlling temperature and environment as separate process steps or at the same time (see discussion below). In yet other disclosures, various milling techniques may be used to separate the bone. For example, an impact mill has blunt rotors, dull knives, or swinging hammers that move at high speed and subdivide the demineralized bone stock by impacting upon the bone, thereby shattering it into fragmentary fibers or particles. The bone tends to shatter along the lines of the natural collagen bundles constituting the microstructure of the bone. Similar mills with sharp cutting rotors tend to chop the bone into somewhat symmetric particles as opposed to the fibrous particles obtained with an impact mill.

A shearing mill subdivides demineralized bone stock by tearing the bone apart. The tearing action tends to preferentially break the bone apart at its weakest point. The junctions between collagen bundles represent weak points and the result is the production of fiber type particles.

In another disclosure, a ball mill may be used to separate the bone. Generally the ball mill may comprise a container with impacting material provided therein. In some embodiments, the container has a surface such that it may be rolled. The impacting material may comprise rollers, irregularly shaped objects, or elements of any suitable shape and size for impacting the bone to separate it. The bone is placed in the container with the impacting material and the container moved, such as by rolling, to cause the bone and impacting material to collide and cause separation of the bone. In some disclosures, a fluid may be provided in the container with the bone and the impacting material. Such fluid may be, for example, a demineralization medium. In some disclosures, the container may be moved by centrifuge force.

In a specific embodiment of separation by pressing the bone, mineralized cortical bone is separated by pressure to form mineralized cortical bone fibers. The mineralized cortical bone fibers may then be demineralized. In some embodiments, a separation step may be done concurrently with demineralization. Such concurrent separation may be preceded by an initial crush or may be followed with a subsequent crush. Generally, an initial crush opens acid entry routes in the bone while a subsequent crush further particulates the bone.

If the bone is nondemineralized when separated, the separated bone may be demineralized. In some disclosures, the bone may separated a first time in a first separation step, demineralized, and separated a second time in a second separation step. In such disclosures, the second separation may be used to obtain desired fibers. The first separation step and the second separation step may use the same or different separation technique. In some disclosures, the bone may be subdivided into strips, the strips may be demineralized, and the demineralized strips may be separated into fibers.

### IV. DEMINERALIZE THE BONE

The bone may be demineralized in any suitable manner. Demineralization may be done before, after, or during separation. In some disclosures, demineralization is concurrent with separation and the acid exposure time to the bone is decreased. Generally, demineralization removes bone minerals that might otherwise interfere with the activity of growth factors. Demineralization further may liberate bone inducing/facilitating proteins such that, when implanted, the bone may induce and facilitate bone growth. Various methods for demineralization are disclosed herein, some including treating the demineralization medium or demineralizing at low temperatures. It is to be appreciated that these methods may not be used in all disclosures. For example, in disclosures wherein separation and demineralization are concurrent, the demineralization medium may not be further treated.

In various embodiments, methods are provided that may optimize or enhance demineralization. These methods may include treating the demineralization medium. For example, demineralization may be accelerated using physical and chemical forces. For example, gravity forces, vacuum, sonication, centrifugation, and other agitation or acceleration techniques may be used. Generally, agitation techniques may be referred to as controlled agitation.

In some disclosures, demineralization may be done at less than room temperature. For example, demineralization may be done at less than approximately 20°C (70°F), less than approximately 18°C (65°F), less than approximately 15°C (60°F), less than approximately 13°C (55°F), less than approximately 10°C (50°F), etc., down to subfreezing temperatures, such as, for example in the presence of salts, or oscillating temperatures.

DBM preparations have been used for many years in orthopedic medicine to promote the formation of bone. For example, DBM has found use in the repair of fractures, in the fusion of vertebrae, in joint replacement surgery, and in treating bone destruction due to underlying disease such as a bone tumor. DBM is has been shown to promote bone formation *in vivo* by osteoconductive and osteoinductive processes. The osteoinductive effect of implanted DBM compositions results from the presence of active growth factors present on the isolated collagen-based matrix. These factors include members of the TGF-β, IGF, and BMP protein families. Particular examples of osteoinductive factors include TGF-β, IGF-1, IGF-2, BMP-2, BMP-7, parathyroid hormone (PTH), and angiogenic factors. Other osteoinductive factors such as osteocalcin and osteopontin are also likely to be present in DBM preparations as well. There are also likely to be other unnamed or undiscovered osteoinductive factors present in DBM.

Generally, demineralization procedures remove the inorganic mineral component of bone by employing acid solutions. Such procedures are well known in the art, see for example, Reddi et al., Proceeding of the National Academy of Sciences of the United States of America 69, pp.1601-1605 (1972). The strength of the acid solution, the shape and size of the bone and the duration of the demineralization procedure influence the extent of demineralization. Generally speaking larger bone portions as compared to small particles require more lengthy and vigorous demineralization. For example, using methods as disclosed herein, small fibers may be demineralized within approximately 3 hours while large fibers may be demineralized within approximately 4 hours. Guidance for specific parameters for the demineralization of different size bone can be found in U.S. Pat. No. 5,846,484, Harakas, Clinical Orthopaedics and Related Research, pp 239-251(1983) and Lewandrowski et al., Journal of Biomedical Materials Research, 31, pp. 365-372 (1996).

Before or after an optional defatting step, the bone is demineralized in accordance with disclosures described herein. In one disclosure, demineralization is performed using acid demineralization and an acceleration technique. For example, demineralization may be performed by placing the bone in a demineralization medium (which may be an acid bath). Generally, acid is provided in a container and the bone is placed in the acid in the container. In some disclosures, the container may be configured to accelerate demineralization, for example, by providing baffles in the container. Suitable demineralization mediums include hydrochloric acid, citric acid, organic acids such as formic acid, acetic acid, peracetic acid, citric acid, propionic acid, or other medium. The depth of demineralization into the bone surface can be controlled by adjusting the treatment time, temperature of the demineralizing solution, concentration of the demineralizing solution, and agitation intensity during treatment. Demineralization can be done to reduce the inorganic content to any desired level. In some disclosures, relatively higher levels of residual mineral content may be maintained after demineralization such that the bone is partially demineralized, surface demineralized, or generally less than fully demineralized.

In various disclosures, the demineralization medium, such as an acid, may be treated while the bone is in the demineralization medium, such as, for example, by reducing the temperature of the demineralization medium and/or increasing the rate of demineralization. As is discussed more fully below, treating the demineralization medium may comprise applying cyclic pressure to the demineralization medium, applying a vacuum to the demineralization medium, stirring the demineralization medium, or otherwise treating the demineralization medium. Such treatment may in some disclosures be useful for reducing regional depletion during demineralization.

Demineralization is accelerated by using vacuum (with or without applying pressure). One example of suitable pressure is the application of cyclic pressure, which may include agitating the demineralization medium with bone therein, for example by stirring, by placing the bone in the demineralization medium under vacuum and/or under pressure, by centrifuging the bone in the demineralization medium, by sonication, pressure waves, or other agitation techniques. In embodiments where the bone is placed in the demineralization medium under vacuum (with or without the application of pressure), other agitation techniques may be used during the vacuum (with or without pressure) and/or during breaks in a vacuum or pressure cycle. Thus, for example, the demineralization medium, under vacuum (with or without pressure), may be stirred, ultrasonication may be performed, and/or other. Vacuum (with or without pressure) may be applied to the liquid that surrounds the bone, or to air adjacent the liquid. Vacuum (with or without pressure) may be applied and then released, once or multiple times, in any desired sequence. The acid solution may be changed during demineralization depending on the duration of demineralization. For example, the acid may be changed if the pH of the acid exceeds 2.0. Application of the teachings herein also may allow for demineralization with acid of higher pH, because the circulation of the acid allows for more efficient application of the acid and thus of demineralization.

In some disclosures, the acid bath may be stirred during demineralization. Stirring may be done, for example, by mechanically stirring the acid with a stir bar. In some embodiments, stirring may be done in a mixing vessel having baffles. In some disclosures, stirring may be done using a ball mill or by mechanically moving a vessel or container. In some disclosures, stirring may be done to acid under vacuum (with or without pressure), including cyclic vacuum (with or without pressure). In some disclosures, perfusion demineralization, which circulates acids by pressure via pumping, may be used.

In some embodiments, a vacuum may be applied during demineralization. Frequently, regional depletion occurs during demineralization. Because the vacuum (or the other techniques taught herein) helps circulate the acid, using a vacuum during demineralization can mitigate problems associated with regional depletion. Using a vacuum during demineralization further reduces the temperature of the demineralization medium. At a reduced temperature of demineralization, there is an increased likelihood that osteoinductive factors will remain in their natural configuration.

In various disclosures, a vacuum may be pulled to between approximately 62.5 kPa (18.5" Hg) and approximately 91.3 kPa (27" Hg). Generally, the vacuum may be pulled for any amount of time that confers the desired benefits. In some disclosures, by way of example only, the vacuum may be pulled and held at a level of above approximately 84.5 kPa (25" Hg). In one embodiment, a vacuum is pulled to approximately 91.3 kPa (27" Hg) and held for approximately an hour with stirring of the acid bath during the hour. The acid may be changed after an hour and the process repeated. The vacuum may be combined with ultrasonic treatment. In some disclosures, ultrasonic treatment of the acid may be done for approximately 10 minutes after the vacuum is released. Accordingly, in one disclosure, a demineralization process may include 5 hours of vacuum and 50 minutes of ultrasonication wherein each hour of vacuum is followed by 10 minutes of ultrasonication. In certain disclosures, any vacuum sufficient to ameliorate depletion zones around the tissue may be used.

The vacuum is pulsed. For example, in one embodiment, a vacuum may be pulled, for example to approximately 62.5Kpa (18.5" Hg) to approximately 64.2 kPa (19.0" Hg), and held for about 1 minute followed by about 3 minutes where the vacuum is released, with this being repeated for, for example, about 10 cycles of vacuum pull followed by vacuum break. In a further example, a vacuum cycle may comprise pulling a vacuum for approximately 10 minutes followed by releasing the vacuum for approximately 2 minutes. In one disclosure, a vacuum is pulled to approximately 64.2 kPa (19.0" Hg) and held for approximately 10 minutes. In another disclosure, a vacuum is pulled for approximately 8 minutes and released for approximately 2 minutes with the cycle being repeated 6 times.

Vacuum demineralization further provides indication of termination of the demineralization process. More specifically, during demineralization, gases (for example CO₂) may form as a result of the reaction of acid and minerals. These gases may form bubbles in the demineralization medium. As used herein, this process (ie formation of gases and/or bubbles) may be referred to as outgassing. When bubbles cease forming, demineralization may be considered complete. In some disclosures, manual or an automated means for observing bubbles or for measuring gas creation may be employed such that observation of a cessation of bubbles or a decrease in gas creation causes an automated shut off of the demineralization process. In various disclosures complete demineralization may be where the calcium content is below 5%, below 1%, below 0.1%, below 0.01%, below 0.005%, below about 0.003%, or about 0.0026%. Demineralization may be measured using a variety of methods, including inductively coupled plasma-mass spectrometry (ICP-MS).

In some disclosures, manual, visual, or automated means for observing bubbles or for measuring gas creation may be employed such that observation of a cessation of bubbles or a decrease in gas creation causes an automated shut off of the demineralization cycle or process.

In some disclosures, manual or an automated means for observing bubbles or for measuring gas creation may be employed such that observation of a cessation of bubbles or a decrease in gas creation causes an automated shut off of the demineralization process.

In some embodiments, demineralization may be done in a centrifuge. Thus, for example, the acid and bone may be provided in a vessel and the vessel may be placed in a centrifuge. The centrifuge may be run at any force that achieves the desired results, for example at 5000 RPM, or at approximately 3000 x the g-force on the centrifuge.

Accordingly, in various embodiments, methods may be used for accelerating the demineralization process. The methods may include vacuum, centrifuge, baffled vessels, ultrasonication, or other. Generally, these accelerate diffusion and penetration of the demineralization medium into the structure of the bone. This, in turn, expedites the demineralization time.

In some disclosures, the bone may be separated into strips, the strips partially demineralized, the partially demineralized strips separated into fibers (such as by pressing), and the fibers demineralized. Accordingly, methods as provided herein may comprise a first separation step, a first demineralization step, a second separation step, and a second demineralization step.

After acid treatment, the demineralized bone is rinsed with sterile water for injection or sterile purified water to remove residual amounts of acid.

### V. COMBINED SEPARATION AND DEMINERALIZATION

In some disclosures, demineralized bone fibers may be formed with substantially contemporaneous separation and demineralization. This step can be performed after a prior partial demineralization step, or it may be performed on previously nondemineralized bone. Further, this step can be performed after an initial crush, or it may be performed on previously un-crushed bone. In one embodiment, mineralized bone may be placed in a demineralization medium and force may be applied to the demineralization medium. The level and duration of the force may be selected such that the force breaks the bone into fibers during the process of demineralization. The level of force may vary depending on the size of the bone and the prior processing of the bone. Thus, any suitable level of force may be used so long as it is sufficient for effecting separation of the bone.

The force may be applied in any suitable manner. In some embodiments, the force may be applied using centrifugal means. In other embodiments, the force may be imparted by a press or a roller. In yet further disclosures, the force may be imparted using hammers, punch presses, spring loaded devices, or similar structure. The force may be a shearing force such as a centrifugal ball mill, a tearing force (such as by scissors, scalpels, or other cutting instruments), or a rasping or grating force (such as by a grating device), or a chipping force. Other forces within the ordinary skill in the art may be applied. Such forces applied to the bone may be done manually, or in an automated or industrial process.

In one disclosure, combined separation and demineralization comprises demineralization with an applied vacuum and separation by pressing. In some embodiments, a pressing force may be applied before demineralization to open acid entry routes in the bone.

### VI. PRE-TREATMENT

After the bone is obtained from the donor, it is processed, e.g., cleaned, disinfected, and optionally defatted, using methods known in the art.

The bone may be further pre-treated to facilitate separation. For example, critical point drying may be performed to remove lipids from bone materials. In some disclosures, combined critical point drying (CPD) and supercritical carbon dioxide (CO₂) treatment may be used on the bone prior to demineralization. Critical point drying protects the delicate surface morphologies of the tissue. U.S. Patent Application 12/140,062 for Method of Treating Tissue teaches suitable CPD and supercritical CO₂ treatment methods.

In some disclosures, pre-treatment of bone involves the use of critical or supercritical fluids to remove lipids and water from the bone. The method may be used for sterilization and/or drying and may be used in lieu of alternative drying processes. Air-drying typically damages tissue samples because very large surface tension forces are created when there is a liquid/gas interface. Similarly, lyophilization of tissue samples can destroy structures by ice formation and removal both at interior and exterior sites. Especially in cases of drying collagen-based tissues such as bone, air drying or lyophilization generally cause deformation and structure collapse. Drying using critical point fluids, as provided herein, avoids these effects by substantially preventing development of a liquid/gas interface. Without such interface, the bone is not exposed to surface tension forces. In some disclosures, critical point drying of bone uses carbon dioxide. Carbon dioxide has relatively low critical point at 31.1°C (304K) with corresponding pressure of 7.58 MPa (1100psi), which is relatively easy to reach and is compatible with biological materials. CO₂ is an excellent non-polar solvent which solubilize lipids, oil and fats in the materials. Above critical point, CO₂ penetrate substantially throughout the material to remove lipidic components.

In a specific disclosure, pre-treatment may comprise dehydrating the bone with a CO₂ miscible solvent, placing the bone in a chamber, flushing the bone to remove the solvent, and drying the bone using critical point drying. Dehydrating the bone with a CO₂ miscible solvent may comprise, for example, treating the bone with a series of graded ethanol solutions (e.g., about 70%, 80%, 90%, 95%, 100% ethanol in deionized water). The dehydrated bone may then be placed in a chamber within a CPD apparatus and flushed with liquid CO₂ several times to remove the CO₂ miscible solvent. Drying the bone using CPD comprises filling the chamber with CO₂, raising the temperature and pressure to the critical point, and then raising the temperature and/or pressure above the critical point. After such treatment, the CO₂ is released. In some disclosures, release of the CO₂ is done slowly, for example at a rate of approximately 0.689 MPa/min (100psi/min). The release rate of CO₂ after treatment may be selected to ensure drying and protecting the microstructures. While this disclosure specifically refers to critical point CO₂ drying, it is to be appreciated that it may be used with other critical point fluids that may be achieved at temperatures and pressures suitable for biologic tissues.

Pre-treatment may be done before separation regardless of whether separation is done before or after demineralization. This CPD pre-treatment may yield bone that has some surfaces that were prepared by CPD, and others by the separation process. These surfaces may have different surface morphologies. Alternatively, the bone can be separated first and then treated with CPD, in contexts where more uniform surface morphologies are desired.

### VII. POST-TREATMENT

If desired, the demineralized bone particles or fibers can be modified in one or more ways, e.g., their protein content can be augmented or modified as described in U.S. Pat. Nos. 4,743,259 and 4,902,296. Any of a variety of medically and/or surgically useful substances can be incorporated in or associated with the bone particles or fibers either before, during or after their formation. For example, one or more of substances can be introduced into the demineralized bone particles or fibers such as by soaking or immersing the bone particles or fibers in a solution or dispersion of the desired substance(s). Bioactive agents and bioactive compounds, as listed above, may be combined with the demineralized bone particles or fibers.

### VIII. CARRIER

The resulting bone particles or fibers can be adjusted to a broad size range and used for creating any suitable bone implant formulation. The matrix structures and protein (growth factor) activities generally are well maintained through the separation and demineralization processes. After creation (including separation and demineralization), depending upon their intended final usage, the demineralized bone particles can be utilized as is or stored under aseptic conditions, including in a lyophilized or frozen state, for use at a later time. In other disclosures, the bone fibers may be added to a carrier to facilitate in vivo use and/or to form an osteoimplant.

A matrix comprising the demineralized bone fibers may be completely insoluble or may be slowly solubilized after implantation. Following implantation, matrices formed in accordance with the teachings herein may resorb or degrade, remaining substantially intact for at least one to seven days, most preferably for two or four weeks or longer and often longer than 60 days. Bioactive agents may be endogenously present in the matrix as in the case of most demineralized bone, or they may be exogenously added to the matrix. Matrices may also comprise combinations of endogenous and exogenous bioactive agents.

The matrix may comprise a number of materials in combination, some or all of which may be in the form of fibers and/or particles. The matrix may comprise calcium phosphates. Driessens et al. "Calcium phosphate bone cements," Wise, D.L., Ed., Encyclopedic Handbook of Biomaterials and Bioengineering, Part B, Applications New York: Marcel Decker; Elliott, Structure and Chemistry of the Apatites and Other Calcium Phosphates Elsevier, Amsterdam, 1994. Calcium phosphate matrices include, but are not limited to, dicalcium phosphate dihydrate, monetite, tricalcium phospate, tetracalcium phosphate, hydroxyapatite, nanocrystalline hydroxyapatite, poorly crystalline hydroxyapatite, substituted hydroxyapatite, and calcium deficient hydroxyapatites. In some disclosures, the bone fibers may be added to a carrier. Generally, materials for the carrier may be biocompatible in vivo and optionally biodegradable. In some uses, the carrier acts as a temporary scaffold until replaced completely by new bone. Suitable carriers include collagen; synthetic hydroxyapatites; polymers; hydrogels; starches; polyethylene glycol, tricalcium phosphate, sintered hydroxyapatite, settable hydroxyapatite; polylactic acid; tyrosine polycarbonate; calcium sulfate; collagen sheets; settable calcium phosphate; polymeric cements; settable poly vinyl alcohols, polyurethanes; resorbable polymers; polysaccharides and other large polymers; liquid settable polymers; and other biocompatible settable materials. Polylactic acid (PLA), polyglycolic acid (PGA), and various combinations have different dissolution rates in vivo. In bone, the dissolution rates can vary according to whether the implant is placed in cortical or trabecular bone. Settable materials may be used, and they may set up either in situ, or prior to implantation. The bone fibers and carrier (or delivery or support system) together form an osteoimplant useful in clinical applications.

Any suitable shape, size, and porosity of carrier may be used. In some disclosures, the carrier may be settable and/or injectable. Such carrier may be, for example, a polymeric cement, a settable calcium phosphate, a settable poly vinyl alcohol, a polyurethane, or a liquid settable polymer. Suitable settable calcium phosphates are disclosed in U.S. Patent Nos. 5,336,264 and 6,953,594.

Further, biocomposites may be used. Suitable materials for preparing biocomposites are disclosed in U.S. Patent Publication Nos. 2007/0191963, 2006/0216323, and 2005/0251267, U.S. Patents Nos. 6,696,073, 6,478,825, 6,440,444, and 6,294,187.

The carrier may comprise a shape-retaining solid made of loosely adhered particulate material, e.g., with collagen. It may also comprise a molded, porous solid, or simply an aggregation of close-packed particles held in place by surrounding tissue. See, e.g., U.S. Patent Nos. 6,863,694, 6,843,807, 6,808,585, 6,294,041, 6,123,731, and 5,899,939. Masticated muscle or other tissue may also be used. Large allogenic bone implants may act as a carrier if their marrow cavities are cleaned and packed with particles and the osteoinductive factors.

### IX. OSTEOIMPLANT

Demineralized bone particles and fibers as disclosed herein find use as, or in, implants, for a variety of orthopedic procedures where they participate in the bone healing/repair process through one or more mechanisms such as osteogenesis, osteoinduction and osteoconduction. The demineralized bone particles or fibers can be used as is, or formed into a variety of product types such as a gel, putty, or sheet. The demineralized bone particles, with or without carrier, can optionally be admixed with one or more substances such as adhesives, fillers, plasticizers, flexibilizing agents, biostatic/biocidal agents, surface active agents, binding and bonding agents, and the like, prior to, during, or after shaping the particles into a desired configuration. Suitable adhesives, binding agents and bonding agents include acrylic resins, cellulosics, bioresorbable polymers such as polyesters, polycarbonates, polyarylates and polyfomarates. Specifically, tyrsine, polycarbonates, tyrosine polyarylates, polyglycolides, polylactides, glycolide-lactide copolymer, etc. Suitable fillers include bone powder, demineralized bone powder, hydroxyapatite, etc. Suitable plasticizers and flexibilizing agents include liquid polyhydroxy compounds such as glycerol, monacetin, diacetin, etc. Suitable biostatic/biocidal agents include antibiotics, providone, sugars, etc. Suitable surface-active agents include the biocompatible nonionic, cationic, anionic and amphoteric surfactants. The bone particles or fibers may be combined with a polymer to form a biocomposite. Suitable materials for preparing biocomposites are disclosed in U.S. Patent Publication Nos. 2007/0191963, 2006/0216323, and 2005/0251267, U.S. Patents Nos. 6,696,073, 6,478,825, 6,440,444, and 6,294,187. U.S. Patent Nos. 7,323,193, 7,163,691, 6,863,694, 6,808,585. 6,616,698, 6,599,520, 6,436,138, 5,676,146, 5,510,396, 5,507,813, 5,484,601, 5,439,684, 5,405,390, 5,314,476, 5,298,254, 5,290,558, 5,284,655, 5,236,456, 5,073,373, U.S. Patent Application Publications Nos. 2007/0098756, 2007/0110820; 2007/0154563; 2009/0130173, and 2009/0192474 and U.S. Patent Application Nos. 12/171,168; 12/205,539; 12/140,062; 12/267,985; 12/140,025; 12/267,985; 12/254,619; 61/108,350, 61/152,057; 61/154,673; 61/154,679, and 61/154,689.

The demineralized bone particles or fibers thus may be subjected to a configuring step to form an implant. The configuring step can be employed using conventional equipment known to those skilled in the art to produce a wide variety of geometries, *e.g.,* concave or convex surfaces, stepped surfaces, cylindrical dowels, wedges, blocks, screws, and the like. A surgically implantable material fabricated from elongated bone particles that have been demineralized, which may be shaped as a sheet, and processes for fabricating shaped materials from demineralized bone particles are disclosed in U.S. Patent Nos. 5,507,813 and 6,436,138, respectively. Suitable sheets include those sold under the trade name Grafton® DBM Flex, which must be wetted/hydrated prior to use to be useful for implantation. Such sheets have recently been reported as effective in seeding human bone marrow stromal cells (BMSCs), which may be useful in the repair of large bone defects. Kasten et al., "Comparison of Human Bone Marrow Stromal Cells Seeded on Calcium-Deficient Hydroxyapatite, Betatricalcium Phosphate and Demineralized Bone Matrix," Biomaterials, 24(15):2593-603, 2003. Also useful are demineralized bone and other matrix preparations comprising additives or carriers such as binders, fillers, plasticizers, wetting agents, surface active agents, biostatic agents, biocidal agents, and the like. Some exemplary additives and carriers include polyhydroxy compounds, polysaccharides, glycosaminoglycan proteins, nucleic acids, polymers, polaxomers, resins, clays, calcium salts, and/or derivatives thereof.

The osteoimplant resulting from a carrier and the osteoinductive factors may assume a determined or regular form or configuration such as a sheet, plate, disk, tunnel, cone, or tube, to name but a few. Prefabricated geometry may include, but is not be limited to, a crescent apron for single site use, an I-shape to be placed between teeth for intra-bony defects, a rectangular bib for defects involving both the buccal and lingual alveolar ridges, neutralization plates, reconstructive plates, buttress plates, T-buttress plates, spoon plates, clover leaf plates, condylar plates, compression plates, bridge plates, or wave plates. Partial tubular as well as flat plates can be fabricated from the osteoimplant. Such plates may include such conformations as, e.g., concave contoured, bowl shaped, or defect shaped. The osteoimplant can be machined or shaped by any suitable mechanical shaping means. Computerized modeling can provide for the intricately-shaped three-dimensional architecture of an osteoimplant custom-fitted to the bone repair site with great precision.

### DELIVERY SYSTEM

In some disclosures, bone particles or fibers as disclosed herein, with or without carrier, may be provided in a covering to form a delivery system.

U.S. Patent Application Serial No. 12/205,539 for Delivery System Attachment discloses suitable coverings for use with demineralized fibers as provided herein to form a delivery system. Further delivery systems are disclosed in the prior section. Accordingly, in some disclosures, a delivery system is provided comprising a covering and demineralized fibers provided within the covering for delivery to the surgical site. Generally, the covering may be a single or multi-compartment structure capable of at least partially retaining a substance provided therein until the covering is placed at a surgical site. The covering may participate in, control, or otherwise adjust, the release of materials from the demineralized fibers or penetration of the covering by surrounding materials, such as cells or tissues. The covering may include one or more attachment mechanisms for retaining the covering at the surgical site. The attachment mechanism may be a mechanical attachment mechanism, a physical attachment mechanism, a biological attachment mechanism or a chemical attachment mechanism, or may employ combinations of these. The attachment mechanism may be used to attach the covering to skeletal or soft tissue proximate the surgical site.

In some disclosures, the covering may be used for containment of the demineralized fibers. In some disclosures, the covering may be used for maintaining the demineralized fibers in spatial proximity to one another, possibly to provide a synergistic effect. In some disclosures, the delivery system may be used for delivery through a limited opening, such as in minimally invasive surgery or mini-open access.

In accordance with alternative disclosures, other delivery systems may be formed using the demineralized fibers. In one disclosure, a plurality of thin sheets are provided. Each sheet of comprises a carrier admixed with demineralized fibers. These sheets are layered and stacked. Thus, in some disclosures, the osteoimplant is formed as a laminate. A laminate osteoimplant may advantageously be shaped in three dimensions, as in the introduction of a concave surface shape. Further, each layer of the laminate is continuous, without requiring binding of the joints between the pieces.

Assembling the superimposed layers into a strong unitary structure may be accomplished by a variety of means/procedures, e.g., application of known and conventional biologically compatible adhesives such as the cyanoacrylates; epoxy-based compounds, dental resin sealants, dental resin cements, glass ionomer cements, polymethyl methacrylate, gelatin-resorcinol-formaldehyde glues, collagen-based glues, inorganic bonding agents such as zinc phosphate, magnesium phosphate or other phosphate-based cements, zinc carboxylate, etc., and protein-based binders such as fibrin glues and mussel-derived adhesive proteins; the use of mechanical fasteners such as pins, screws, dowels, etc., which can be fabricated from natural or synthetic materials and bioabsorbable as well as nonbioabsorbable materials; laser tissue welding; and ultrasonic bonding. If desired, the layers of the osteogenic osteoimplant can be provided with mechanically interengaging features, e.g., tongue-and-groove, mortise-and-tenon, or dove-tail elements, to facilitate their assembly into the final product and/or to fix the layers to each other in a more secure fashion. The optimal method of assembly would be determined on a case-by-case basis through routine experimentation. In addition to its carrier and osteoinductive layers, the osteoimplant of this disclosure can optionally possess one or more layers formed from one or more other materials or substances.

In another disclosure, the carrier may comprise a single thin sheet of material. The delivery systems thus may comprise a single thin sheet of material comprising carrier and demineralized fibers as disclosed herein. The sheet of material may be rolled or folded over itself.

### FORMULATION

The carrier, the osteoinductive composition, the covering, or the osteoimplant may be formulated for a particular use. The formulation may be used to alter the physical, biological, or chemical properties of any component of the osteoimplant. A physician would readily be able to determine the formulation needed for a particular application, taking into account such factors as the type of injury, the site of injury, the patient's health, and the risk of infection. In various disclosures, the osteoinductive composition may comprise, for example less than approximately 0.5% water, less than approximately 1% water, or less than approximately 5% water.

Carriers, osteoinductive compositions, coverings, or osteoimplants therefore may be prepared to have selected resorption/loss of osteoinductivity rates, or even to have different rates in different portions of an implant. For example, the formulation process may include the selection of demineralized particles or fibers of a particular size or composition, combined with the selection of a particular stabilizing agent or agents, and the amounts of such agents.

Physical properties such as deformability and viscosity of the carrier may also be chosen depending on the particular clinical application. Further, the composition may be formulated to be settable and/or injectable. Thus, for example, the composition may be injectable through a needle, such as a 10-gauge, a 12-gauge, or an 18-gauge needle, as desired.

In the process of preparing the osteoimplant, the materials may be produced entirely aseptically or be sterilized to eliminate any infectious agents such as HIV, hepatitis B, or hepatitis C. The sterilization may be accomplished using antibiotics, irradiation, chemical sterilization (e.g., ethylene oxide), or thermal sterilization. Other methods known in the art of preparing DBM such as defatting, sonication, and lyophilization may also be used in preparing a DBM carrier. Since the biological activity of demineralized bone is known to be detrimentally affected by most terminal sterilization processes, care must be taken when sterilizing the inventive compositions.

### X. OPTIONAL ADDITIVES

Optionally, other additives may be included in an osteoimplant or osteocomposition comprising bone fibers as disclosed herein. It will be appreciated that the amount of additive used will vary depending upon the type of additive, the specific activity of the particular additive preparation employed, and the intended use of the composition. The desired amount is readily determinable by the user. Any of a variety of medically and/or surgically useful optional substances can be incorporated in, or associated with, the osteoinductive factors either before, during, or after preparation of the osteogenic composition.

In certain disclosures, the additive is adsorbed to or otherwise associated with the osteoimplant or osteocomposition. The additive may be associated with the osteoimplant through specific or non-specific interactions, or covalent or noncovalent interactions. Examples of specific interactions include those between a ligand and a receptor, an epitope and an antibody, *etc.* Examples of nonspecific interactions include hydrophobic interactions, electrostatic interactions, magnetic interactions, dipole interactions, van der Waals interactions, hydrogen bonding, *etc.* In certain disclosures, the additive is attached to the osteoimplant, for example, to the carrier, using a linker so that the additive is free to associate with its receptor or site of action *in vivo.* In other disclosures the additive is either covalently or non-covalently attached to the carrier. In certain disclosures, the additive may be attached to a chemical compound such as a peptide that is recognized by the carrier. In another disclosure, the additive is attached to an antibody, or fragment thereof, that recognizes an epitope found within the carrier. In certain disclosures at least additives are attached to the osteoimplant. In other disclosures at least three additives are attached to the osteoimplant. An additive may be provided within the osteoimplant in a sustained release format. Thus, delivery of an osteoactive agent in an osteoimplant may include micro- or nano-encapsulation. For example, the additive may be encapsulated within biodegradable nanospheres, microspheres, *etc.* In this method, an osteoactive agent (or bioactive agent) is first encapsulated in a suitable material, such as a bioresorbably polymer or hydrogel micro- or nano-particle. The encapsulated osteoactive agent then is immobilized onto or into the osteoimplant. The bioresorbably polymer/hydrogel may be designed to control release kinetics of the osteoactive agent.

Generally, bioactive agents including, for example, bone growth promoting factors may be added to compositions of bone formed as described herein. Further, factors that stop, remove, or reduce the activity of bone growth inhibitors may also be added.

It will be understood by those skilled in the art that the lists of optional substances herewith included are not intended to be exhaustive and that other materials may be admixed with bone-derived elements within the practice of the present invention.

Radiopaque substances may be added to impart radiopacity to the composition. Examples of substances imparting radiopacity include for example, fully mineralized bone particles, Barium and Iodine containing compounds or compositions, e.g., Barium Sulfate and Barium Sulfate for Suspension, lopanoic Acid, and the like. Mineralized cancellous bone, mineralized cortical bone, and partially demineralized bone, in solid, particle or fiber form may be added to endow the material with radiopacity. When employed, substances imparting radiopacity will typically represent from about 1 to about 25 weight percent of the bone particle containing composition, calculated prior to forming the shaped material. U.S. Patent No. 5,676,146 discusses radiopaque bone grafts.

Development of a vasculature around the implant site may also be important to forming new bone and/or cartilage tissues. Angiogenesis may be an important contributing factor for the replacement of new bone and cartilage tissues. In certain disclosures, angiogenesis is promoted so that blood vessels are formed at the site to allow efficient transport of oxygen and other nutrients and growth factors to the developing bone or cartilage tissue. Thus, angiogenesis promoting factors may be included in the osteoimplant to increase angiogenesis in that region. For example, class 3 semaphorins, *e.g.*, SEMA3, controls vascular morphogenesis by inhibiting integrin function in the vascular system, Serini et al., Nature, (July 2003) 424:391-397, and may be included in the osteoimplant. Vascular Endothelial Growth Factor (VEGF) and other cystine-knot growth factors may be used as well.

The osteoconductive composition may provide a system for delivering bioactive agents, such as osteoinductive factors, to a host animal. Thus, the osteoimplant enables an improved healing response to the implant without the need to administer separately the bioactive agent. A problem with the introduction of the bioactive agent at the site is that it is often diluted and redistributed during the healing process by the circulatory systems (e.g., blood, lymph) of the recipient before complete healing has occurred. A solution to this problem of redistribution is to affix the bioactive components, including osteoactive agents, to the osteoimplant. Suitable bioactive agents and bioactive compounds are listed in the definition section of this application. Some preferred bioactive agents that can be delivered using a DBM composition include agents that promote the natural healing process, i.e., resorption, vascularization, angiogenesis, new growth, etc. In one disclosure, the osteoimplant is provided in which DBM, together with a stabilizing agent, is used to deliver the biologically active agent. It is expected that the stabilizing agent will protect the biologically active agent from degradation, and therefore will extend its active life after delivery into the recipient animal. In certain disclosures, the bioactive agent is an osteoinductive agent, and in certain disclosures, the DBM may be used to deliver more than one bioactive agent, preferably more than two, and more preferably sometimes more than three bioactive agents. The bioactive agent may be associated with the DBM. For example, the bioactive agent may be associated with the DBM through electrostatic interactions, hydrogen bonding, pi stacking, hydrophobic interactions, van der Waals interactions, etc. In certain disclosures, the bioactive agent is attached to the DBM through specific interactions such as those between a receptor and its ligand or between an antibody and its antigen. In other disclosures, the bioactive agent is attached to the DBM through non-specific interactions (e.g., hydrophobic interactions).

Medically/surgically useful substances include physiologically or pharmacologically active substances that act locally or systemically in the host. Generally, these substances may include bioactive substances which can be readily incorporated into the osteoimplant and include, e.g., demineralized bone powder as described in U.S. Pat. No. 5,073,373; collagen, insoluble collagen derivatives, etc., and soluble solids and/or liquids dissolved therein; antiviricides, particularly those effective against HIV and hepatitis; antimicrobials and/or antibiotics such as erythromycin, bacitracin, neomycin, penicillin, polymycin B, tetracyclines, biomycin, chloromycetin, and streptomycins, cefazolin, ampicillin, azactam, tobramycin, clindamycin and gentamycin, etc.; biocidal/biostatic sugars such as dextran, glucose, etc.; amino acids; peptides; vitamins; inorganic elements; co-factors for protein synthesis; hormones; endocrine tissue or tissue fragments; synthesizers; enzymes such as alkaline phosphatase, collagenase, peptidases, oxidases, etc.; polymer cell scaffolds with parenchymal cells; angiogenic agents and polymeric carriers containing such agents; collagen lattices; antigenic agents; cytoskeletal agents; cartilage fragments; living cells such as chondrocytes, bone marrow cells, mesenchymal stem cells; natural extracts; genetically engineered living cells or otherwise modified living cells; expanded or cultured cells; DNA delivered by plasmid, viral vectors or other means; tissue transplants; demineralized bone powder; autogenous tissues such as blood, serum, soft tissue, bone marrow, etc.; bioadhesives; bone morphogenic proteins (BMPs); osteoinductive factor (IFO); fibronectin (FN); endothelial cell growth factor (ECGF); vascular endothelial growth factor (VEGF); cementum attachment extracts (CAE); ketanserin; human growth hormone (HGH); animal growth hormones; epidermal growth factor (EGF); interleukins, e.g., interleukin-1 (IL-1), interleukin-2 (IL-2); human alpha thrombin; transforming growth factor (TGF-beta); insulin-like growth factors (IGF-1, IGF-2); platelet derived growth factors (PDGF); fibroblast growth factors (FGF, BFGF, etc.); periodontal ligament chemotactic factor (PDLGF); enamel matrix proteins; growth and differentiation factors (GDF); hedgehog family of proteins; protein receptor molecules; small peptides derived from growth factors above; bone promoters; cytokines; somatotropin; bone digesters; antitumor agents; cellular attractants and attachment agents; immuno-suppressants; permeation enhancers, e.g., fatty acid esters such as laureate, myristate and stearate monoesters of polyethylene glycol, enamine derivatives, alpha-keto aldehydes, etc.; and nucleic acids. The amounts of such optionally added substances can vary widely with optimum levels being readily determined in a specific case by routine experimentation.

In certain disclosures, the agent to be delivered is adsorbed to or otherwise associated with the osteoimplant. The agent may be associated with the osteoimplant through specific or non-specific interactions; or covalent or non-covalent interactions. Examples of specific interactions include those between a ligand and a receptor, a epitope and an antibody, etc. Examples of non-specific interactions include hydrophobic interactions, electrostatic interactions, magnetic interactions, dipole interactions, van der Waals interactions, hydrogen bonding, etc. In certain disclosures, the agent is attached to the osteoimplant using a linker so that the agent is free to associate with its receptor or site of action in vivo. In certain disclosures, the agent to be delivered may be attached to a chemical compound such as a peptide that is recognized by the matrix of the DBM composition. In another disclosure, the agent to be delivered is attached to an antibody, or fragment thereof, that recognizes an epitope found within the matrix of the DBM composition. In a further disclosure, the agent is a BMP, TGF-β, IGF, parathyroid hormone (PTH), growth factors, or angiogenic factors. In certain disclosures at least two bioactive agents are attached to the DBM composition. In other disclosures at least three bioactive agents are attached to the DBM composition.

### XI. TREATMENT OF COMPOSITIONS

In the process of preparing improved inventive bone and cartilage matrix materials, the materials may be produced entirely aseptically or be sterilized to eliminate any infectious agents including viruses, such as HIV, hepatitis B, or hepatitis C, as well as bacteria, mold, yeast, or other infectious agents. The sterilization may be accomplished using antibiotics, irradiation, chemical sterilization (*e.g.*, ethylene oxide), or thermal sterilization. Other methods known in the art of preparing bone and cartilage matrices, such as defatting, sonication, and lyophilization may also be used in preparing the carrier. Since the biological activity of various materials including demineralized bone is known to be detrimentally affected by most terminal sterilization processes, care must be taken when sterilizing the inventive compositions. In some disclosures, the osteoimplants described herein will be prepared aseptically or sterilized.

### XII. EXAMPLES

In accordance with one example, bone is separated and demineralized using a carver press with stir bars and baffles. The process takes approximately 5 hours to achieve full demineralization of up to 40 grams of bone. Demineralization takes approximately 3.5 hours and Separation takes approximately 1.5 hours.

### Example 1

Several demineralization techniques were used and the rate of demineralization compared.

In a first technique, for comparison purposes, a human allograft bone shaft was demineralized using standard techniques. The bone was cut into 2-5mm thick strips using a band saw. Twenty grams (20g) of bone strips were soaked in 300ml 0.6N HCl solution with 0.075ml Triton X100 with stirring. After 2.5 hours, a fresh 300ml 0.6N HCl solution was replaced and the demineralization was continued for additional 60-72 hours. The completion of demineralization was confirmed with X-ray imaging. After imaging, the demineralized bone strips were rinsed with deionized water three times and were pressed into fibers using a carver press under a pressure of 27.57-34.47 MPa (4000-5000psi). Demineralized bone fibers were collected between a 0.5mm and a 4.0mm sieve for further processing. The total demineralization time for this technique was about 62.5-74.5 hours with 2 acid soaks.

In a second technique (of the present invention), human allograft was demineralized using a technique including applying pressure to the bone during demineralization. A human allograft bone shaft was cut into 2-5mm thick and 25mm long strips using a band saw. About ten grams (10g) of bone strips were soaked in 100ml 0.6N HCl solution with 0.025ml Triton X100 with stirring. After 1 hour, the bone strips was collected and pressed under a pressure of 27.57-34.47 MPa (4000-5000psi). A fresh 100ml 0.6N HCl solution was added and the demineralization was continued for 1 hour with stirring. The press/acid change was repeated 3 times. (It is to be appreciated that the pressing could be done while the bone is in contact with or submerged within the demineralization solution.) After the final press/acid change, the demineralization process was continued for another 3 hours. Demineralized bone fibers were collected between a 0.5mm and a 4.0mm sieve for further processing. The completion of demineralization was confirmed with X-ray imaging. The total demineralization time for this technique was about 8-10 hours with 5 acid changes.

In a third technique (of the present invention), human allograft was demineralized using a demineralization including pulling a vacuum during demineralization and applying pressure to the bone during demineralization. A human allograft bone shaft was cut into 0-5mm thick and 30mm long strips using a band saw. About one hundred and eighty-five grams (184.9g) of bone strips were soaked in 2774ml 0.6N HCl solution under vacuum 94.8-101.6 kPa (28-30 in Hg). After 5 minutes, the vacuum was stopped and fresh acid was added with 2.77ml 25% Triton X100. Demineralization was continued for 10 minutes without vacuum. The bone strips were then pressed using grooved plates. This process was repeated twice. After that, fresh acid was changed and demineralization was continued with vacuum for cycles of: 5 minutes in vacuum, press with flat plates; 30 minutes in vacuum, press with flat plates; 20-60 minutes in vacuum, final press with flat plates. The completion of demineralization was observed when gas output did not occur in the final vacuum. Completion of demineralization was confirmed by calcium content test. The total demineralization time for this process was about 2-4 hours with 5-7 acid changes.

Results of Example 1 are shown in Table 1, below.

| Process | Total time |
|---|---|
| Press after Demineralization, No vacuum - not of the present invention | 60-75 hours |
| Press during demineralization, stirring, multiple cycles, no vacuum | 8-10 hours |
| Press during demineralization, with vacuum, multiple cycles | 2-4 hours |

It was determined that applying pressure to the bone during demineralization and/or pulling a vacuum during demineralization accelerates the demineralization rate.

### Example 2

This example examined the relationship between outgassing during vacuum demineralization (as described in Example 1) and the calcium content of the resulting demineralized bone fibers.

Human allograft bone shaft was cut into 2-5mm thick by 20mm, 30mm, and 40mm long strips using a band saw. Resulting bone strips were weighed ((13.53-13.65g), and then soaked in approximately 136.5ml 0.6N HCl under vacuum while outgassing from bone was monitored. After 5 minutes, vacuum was released and bone strips pressed with grooved plates, then returned to the reactor along with the same volume of fresh acid. This step was repeated once, after which the acid was changed and about 0.14ml 25% Triton was added. After about 5 minutes without vacuum, the bone strips were removed and pressed with grooved plates. The bone strips were placed back in the reactor and the reaction was continued with vacuum for cycles of: 5 minutes in vacuum, press with flat plates; 5 minutes in vacuum, press with flat plates; 30 minutes in vacuum, press with flat plates; 5 minutes in vacuum, press with flat plates; 60 minutes in vacuum, press with flat plates. After above demineralization process (with a total acid time of 120 minutes), acid was replaced with fresh acid and the reaction was continued until no outgassing was observed. The total demineralization time was recorded. In case outgassing was observed to cease in any of the vacuum cycles, the reaction would have been stopped and reaction time recorded. The demineralized bone fiber was washed with deionized water 3 times with 10 minutes per wash. The bone was then dried. Demineralized bone samples were measured for calcium content using Inductively Coupled Plasma-Mass Spectrometry (ICP).

Results from this experiment demonstrate that the progress of demineralization can be followed by monitoring outgassing, as shown in Table 2, below.

| Bone thickness (mm) | Bone length (mm) | Bone weight (g) | Acid demineralization time (outgassing ceasing) (min.) | Calcium content (%) |
|---|---|---|---|---|
| 2-5 | 20 | 13.64 | 130 | 0.0024 |
| 2-5 | 30 | 13.65 | 130 | 0.0026 |
| 2-5 | 40 | 13.53 | 130 | 0.0028 |

### Example 3 (not of the present invention)

This example looked at centrifuge demineralization. A human allograft bone shaft was cut into 2-5mm thick strips using a band saw. The total mass of strips was divided into 4 buckets. The buckets were 2.5 liter and included a custom fixture for sitting the bone strips above the bottom of the bucket. The buckets were closed and placed into a centrifuge (Beckman-Coulter Model J-HC with JS-5.0 Rotor) and the centrifuge for a 10 minute cycle at 7380 x G. At the completion of the cycle, lipids from the bone were removed from the bottom of the bucket. The strips were placed in clean buckets (without fixtures) using enough acid to meet a 300ml 0.6N HCl solution with 0.075ml Triton X100 for every 20 g of bone strips. The buckets were closed and placed into the centrifuge for a 20 minute cycle at 7380 x G. The bone strips were transferred back to buckets having fixtures. The buckets were closed and placed into the centrifuge for a 15 minute cycle at 7380 x G. The bone strips and fixtures were removed and residual fluid on the bottom of the buckets was drained. The bone strips placed on the bottom of each bucket and custom heavy fixtures were placed on top of the bone strips. The heavy fixtures function to apply crushing force to the bone strips. The buckets were closed and placed into the centrifuge for a 15 minute cycle at 7380 x G. At the completion of this cycle, the bone strips were placed back into the acid buckets (without fixtures) with fresh acid and no Triton X-100. The buckets were placed in the centrifuge for a 15 minute cycle at the same conditions. These cycles thus comprise an acid centrifuge cycle, an acid removal centrifuge cycle, and a compression centrifuge cycle. The bone strips were run repeatedly with these cycles until what remained was demineralized fibers as determined by X-ray. Following demineralization, the demineralized bone fibers were soaked in deionized water. Total demineralization took place in about 7 hours.

### Example 4 (not of the present invention)

This example looked at demineralization and subsequent pressing using a roller compactor. A human allograft bone shaft was cut into 2-5mm thick strips using a band saw. Twenty gram (20g) bone strips were soaked in 300ml 0.6N HCl solution with 0.075ml Triton X100 under stirring. After 2.5 hours, the solution was replaced with a fresh 300ml 0.6N HCl solution and demineralization was continued for additional 60-72 hours. Completion of demineralization was confirmed with X-ray imaging. After that, the demineralized bone strips were rinsed with deionized water three times and were pressed into fibers using a roller compactor (e.g. a FitzPatrick Company Chilsonator Model IR220). The demineralized bone strips were fed into the top of moving rollers of the compactor and the fibers either dropped out the bottom of the rollers or were scraped off with roller scrapers. Different roller designs were tested ranging from smooth surfaced rollers and grooved rollers to knurled roller designs. Different forces were applied with a maximum force of 1751.3 n/mm (10 000 lbs per linear inch).

## Claims

1. A method for demineralizing bone comprising:
demineralizing the bone in a demineralization medium,
applying a pulsating vacuum to the demineralization medium during demineralizing, and
separating the bone into particles during demineralization.

2. The method of claim 1, wherein separating is done by applying a force to the bone during demineralization.

3. The method of claim 2, wherein the force is applied using a roller press by feeding the bone through rollers of the roller press;
wherein, optionally, the bone is fed through the rollers in a direction to cause longitudinal separation of the bone.

4. The method of claim 2, wherein the force is applied using a ball mill by placing an impacting material and the bone in the ball mill and moving the ball mill such that the impacting material and the bone collide;
wherein, optionally, the demineralization medium is in the ball mill.

5. The method of claim 3, wherein the press rollers are irregularly shaped.

6. The method of claim 2, wherein the force is applied using pressure crushing and impact crushing.

7. The method of claim 1, wherein separating is done by placing the bone in a centrifuge and centrifuging the bone;
wherein, optionally, centrifuging is done while the bone is being demineralized.

8. The method of claim 1, wherein the particles are fibers.

9. The method of claim 1, further comprising applying a force to the bone before demineralizing to precrush the bone.

10. The method of claim 1, further comprising applying a force to the bone after demineralizing to further separate the bone.

11. The method of claim 1, further comprising agitating the demineralization medium during demineralizing.

12. The method of claim 1, wherein the demineralizing is done at less than room temperature.

13. The method of claim 1, wherein the demineralizing is performed in a vessel with baffles.

14. The method of claim 1, wherein demineralization is monitored by monitoring outgassing.

## Patentansprüche

1. Verfahren zum Demineralisieren von Knochen, umfassend:
Demineralisieren des Knochens in einem Demineralisierungsmedium,
Herstellen eines pulsierenden Vakuums in dem Demineralisierungsmedium während des Demineralisierens und
Trennen des Knochens in Partikel während des Demineralisierens.

2. Verfahren nach Anspruch 1, wobei das Trennen durch Aufbringen einer Kraft auf den Knochen während des Demineralisierens erfolgt.

3. Verfahren nach Anspruch 2, wobei die Kraft unter Verwendung einer Walzenpresse durch Führen des Knochens durch Walzen der Walzenpresse aufgebracht wird;
wobei optional der Knochen in einer Richtung durch die Walzen geführt wird, um eine Längstrennung des Knochens zu bewirken.

4. Verfahren nach Anspruch 2, wobei die Kraft unter Verwendung einer Kugelmühle durch Platzieren eines Schlagmaterials und des Knochens in der Kugelmühle und derartiges Bewegen der Kugelmühle, dass das Schlagmaterial und der Knochen kollidieren, aufgebracht wird;
wobei sich optional das Demineralisierungsmedium in der Kugelmühle befindet.

5. Verfahren nach Anspruch 3, wobei die Presswalzen unregelmäßig geformt sind.

6. Verfahren nach Anspruch 2, wobei die Kraft unter Verwendung einer Druckzerkleinerung und einer Schlagzerkleinerung aufgebracht wird.

7. Verfahren nach Anspruch 1, wobei das Trennen durch Platzieren des Knochens in eine Zentrifuge und Zentrifugieren des Knochens erfolgt;
wobei optional das Zentrifugieren erfolgt, während der Knochen demineralisiert wird.

8. Verfahren nach Anspruch 1, wobei die Partikel Fasern sind.

9. Verfahren nach Anspruch 1, das ferner das Aufbringen einer Kraft auf den Knochen vor dem Demineralisieren umfasst, um den Knochen vorab zu zerkleinern.

10. Verfahren nach Anspruch 1, das ferner das Aufbringen einer Kraft auf den Knochen nach dem Demineralisieren umfasst, um den Knochen weiter zu trennen.

11. Verfahren nach Anspruch 1, das ferner ein Hin- und Herbewegen des Demineralisierungsmediums während des Demineralisierens umfasst.

12. Verfahren nach Anspruch 1, wobei das Demineralisieren bei weniger als Raumtemperatur erfolgt.

13. Verfahren nach Anspruch 1, wobei das Demineralisieren in einem Gefäß mit Leitblechen durchgeführt wird.

14. Verfahren nach Anspruch 1, wobei das Demineralisieren durch Überwachen einer Ausgasung überwacht wird.

## Revendications

1. Procédé de déminéralisation de l'os comprenant :
la déminéralisation de l'os dans un milieu de déminéralisation,
l'application d'un vide pulsé au milieu de déminéralisation pendant la déminéralisation, et
la séparation de l'os en particules lors de la déminéralisation.

2. Procédé selon la revendication 1, dans lequel la séparation est effectuée par application d'une force à l'os pendant la déminéralisation.

3. Procédé selon la revendication 2, dans lequel la force est appliquée à l'aide d'une presse à rouleaux par passage de l'os à travers des rouleaux de la presse à rouleaux ;
dans lequel, éventuellement, l'os passe à travers les rouleaux dans une direction pour provoquer une séparation longitudinale de l'os.

4. Procédé selon la revendication 2, dans lequel la force est appliquée à l'aide d'un broyeur à boulets par placement d'une matière d'impact et de l'os dans le broyeur à boulets et par déplacement du broyeur à boulets de telle sorte que la matière d'impact et l'os entrent en collision ;
dans lequel, éventuellement, le milieu de déminéralisation est dans le broyeur à boulets.

5. Procédé selon la revendication 3, dans lequel les rouleaux presseurs sont de forme irrégulière.

6. Procédé selon la revendication 2, dans lequel la force est appliquée à l'aide d'un concassage par pression et d'un concassage par impact.

7. Procédé selon la revendication 1, dans lequel la séparation est effectuée par placement de l'os dans une centrifugeuse et par centrifugation de l'os ;
dans lequel, éventuellement, la centrifugation est effectuée pendant que l'os est en cours de déminéralisation.

8. Procédé selon la revendication 1, dans lequel les particules sont des fibres.

9. Procédé selon la revendication 1, comprenant en outre l'application d'une force à l'os avant la déminéralisation pour préconcasser l'os.

10. Procédé selon la revendication 1, comprenant en outre l'application d'une force à l'os après la déminéralisation pour séparer davantage l'os.

11. Procédé selon la revendication 1, comprenant en outre l'agitation du milieu de déminéralisation pendant la déminéralisation.

12. Procédé selon la revendication 1, dans lequel la déminéralisation est effectuée à une température inférieure à la température ambiante.

13. Procédé selon la revendication 1, dans lequel la déminéralisation est effectuée dans un récipient pourvu de chicanes.

14. Procédé selon la revendication 1, dans lequel la déminéralisation est surveillée par surveillance du dégazage.
